# EUROPÄISCHE PATENTANMELDUNG

(11) **EP 3 269 375 A1**
(43) Veröffentlichungstag der Anmeldung: **17.01.2018**
(21) Anmeldenummer: 17179987.7
(22) Anmeldetag: 06.07.2017
(51) Int. Cl.: A61K 31/7068, A61P 35/00

(54) **KREBSMEDIKAMENT**

(30) Priorität: 15.07.2016 DE 102016113143
(71) Anmelder: Pallua, Christoph, 6020 Innsbruck (AT)
(72) Erfinder: Pallua, Christoph, 6020 Innsbruck (AT)
(74) Vertreter: LS-MP von Puttkamer Berngruber Loth Spuhler

(57) **Zusammenfassung**

Ein Krebsmedikament wird durch eine Pyrimidin-Base mit in N-1-Postion glykosidisch gebundener Glucose oder Gluconsäure gebildet, wobei die Pyrimdin-Base in ihrer 5-Position mit einer Furfural-Gruppe substituiert ist.

## Beschreibung

Die Erfindung bezieht sich auf ein Krebsmedikament, das ein Pyrimidin-Derivat enthält, das durch eine Pyrimidin-Base mit einem in N-1-Position glykosidisch gebundenen Zuckerderivat gebildet wird und eine Furfural-Gruppe aufweist.

Im Gegensatz zur gesunden Zelle, welche einen metabolischen Stoffwechsel mit Sauerstoff aufweist, weist die Krebszelle einen Gärungsstoffwechsel auf.

Durch den Gärungsstoffwechsel werden Wasserstoff-Ionen gebildet, die zur Übersäuerung und damit zur Zerstörung der Krebszelle führen. Gegen diese Übersäuerung schützt sich die Krebszelle jedoch durch reduktive Aminierung, durch die die Wasserstoff-Ionen und Stickstoff gebunden werden.

Ein Stoff, der die reduktive Aminierung blockiert, würde damit zur Übersäuerung und demzufolge zum Zelltod der Krebszelle führen.

Es ist bereits vorgeschlagen worden, die reduktive Aminierung der Krebszelle durch Verabreichung von Furfural zu blockieren, um eine Übersäuerung und damit den Zelltod der Krebszelle herbeizuführen (vergleiche Dr. Dr. Rudolph Dobril "Schluckimpfung" gegen Krebs, Verlag Wilhelm Maudrich, Wien-München-Bern, zweite Auflage 1985, insbesondere seit 39 bis 61).

Wie festgestellt worden ist, hält der Blutspiegel von Furfural jedoch nur kurze an, sodass die Wirksamkeit von Furfural als Krebsmedikament zu gering ist, insbesondere bei großen metastasierenden Tumoren.

Um die reduktive Aminierung der Krebszelle wirksamer zu blockieren, ist bereits ein Ester aus Gluconsäure und Hydroxylmethyl-Furfural bekannt (DE 10 2013 106530 A1).

Ferner ist es bekannt, N(4)-Pentyloxycarbonyl-5'-Desoxy-5-fluorcytidin (INN-Bezeichnung: Capecitabin) zur Therapie von Dickdarmkrebs und fortgeschrittenem Mammakarzinom einzusetzen. Capecitabin wird in der Leber zu 5'- Desoxy-5-fluorcytidin hydrolisiert, das in Zellen zu 5'-Desoxy-5-flururidin umgewandelt und durch das Enzym Thymidinphosphorylase, das im Tumorgewebe in besonders hoher Konzentration auftritt, zu dem Wirkstoff- 5-Fluoruracil abgebaut wird.

Damit werden zwar bestimmte Nebenwirkungen, wie Haarausfall, seltener beobachtet, als wenn 5- Fluoruracil direkt verabreicht wird; dafür treten eine Reihe weiterer zum Teil schwerer Nebenwirkungen häufiger auf.

Aus DE 10 2014 113 936 A1 ist es bekannt, als Krebsmedikament ein in der 5'-Postition des Ribose-Restes mit einer Furfural-Gruppe substituiertes Pyrimidin-Nucleosid, z.B. Uridin, zu verwenden.

Aus WO 2011/051733 A2 geht ein Verfahren hervor, mit dem die Bindungsaffinität von 5-(5-Formylfuran-2yl)-uridin-5'-monophosphat an Glycosyl-transferase-Protein bestimmt werden kann.

Aufgabe der Erfindung ist es, ein wirksames Krebsmedikament bereit zu stellen, das durch gezielte Ausrichtung des Wirkmechanismus für Patienten besser verträglich ist.

Dies wird erfindungsgemäß durch das in Anspruch 1 gekennzeichnete Krebsmedikament erreicht, das ein Pyrimidin-Derivat enthält, das durch eine Pyrimidin-Base mit in N-1-Position glykosidisch gebundener Glucose oder Gluconsäure oder einem Glucose- oder Gulconsäure-Derivat gebildet wird und wenigstens eine Furfural-Gruppe aufweist, wobei die Glucose oder die Gluconsäure bzw. deren Derivat vorzugsweise glucosidisch, also insbesondere in der 1'-Position an die N-1-Position der Primidin-Base gebunden ist.

Wie in DE 10 2013 106 530 A1 beschrieben, werden Glucose und Gluconsäure durch den Gärungsstoffwechsel der Krebszelle rasch metabolisiert. Durch die Freisetzung der Glucose bzw. Gluconsäure und deren Derivate durch die im Tumorgewebe in hoher Konzentration auftretende Thymidinphosphorylase und die rasche Metabolisierung der Glucose bzw. Gluconsäure und deren Derivate wird die Aufnahme der mit der Furfural-Gruppe substituierten Pyrimidin-Base in der Krebszelle beschleunigt und somit die Wirksamkeit des erfindungsgemäßen Medikaments wesentlich erhöht.

Damit wird in das Tumorgewebe ein Krebsmedikament mit langanhaltender toxischer Wirkung für die Krebszellen eingebracht.

Vorzugsweise ist die Pyrimidin-Base in ihrer 5-Position mit einer weiteren Furfural-Gruppe substituiert. Dabei kann die Furfural-Gruppe direkt an die Pyrimidin-Base oder über eine Ether-, Ester, oder Alkylgruppe mit der Furfural-Gruppe verbunden sein.

Die Pyrimidin-Base kann Uracil, Cytosin oder Thymin sein.

Die Furfural-Gruppe kann an die Pyrimidin-Base und/oder Gluconsäure gebunden sein.

So kann zur Synthese der mit der Furfural-Gruppe substituierten Pyrimidin-Base z.B. von 5-Fluor (oder Jod)-Cytosin oder -Uracil ausgegangen werden, das mittels Williamson-Ethersynthese mit dem Alkoholat von 5-Hydroxy-methyl-furfural in Cytosin oder Uracil umgesetzt wird, welches in der 5-Position durch Oxymethyl-Furfural substituiert ist. Auch kann das Oxymethyl-Furfural substituierte Pyrimidin bzw. Uracil oder Cytosin unter Verwendung von Silberjodid aus 5-Hydroxy-Furfural und 5-Fluor- oder 5-Jod-Uracil nach Williamson hergestellt werden (vgl. DE 10 2014 113 936 A1).

Außer der an die Pryimidin-Base gebundenen Furfural-Gruppe oder zusätzlich zu dieser insbesondere an Cytosin oder Uracil in der 5-Position als Oxymethyl-Furfural gebundene Fufural-Gruppe weist das erfindungsgemäße Krebsmedikament vorzugsweise eine weitere an Gluconsäure gebundene Furfural-Gruppe auf.

Die Gluconsäure ist dabei mit ihrer 1'-Position an die N-1-Position der Pyrimidin-Base gebunden und mit ihrer Carboxyl-Gruppe mit Hydroxymethyl-Furfural verestert, wobei der Ester der Gluconsäure mit Hydroxymethyl-Furfural entsprechend DE 10 2013 106 530 A1 z.B. durch Umsetzung des Silbersalzes der Gluconsäure mit Jodmethyl-Furfural unter Abspaltung von Silberjodid hergestellt werden kann.

Die Herstellung des erfindungsgemäßen Krebsmedikaments kann analog zu dem in US 2011/0224422 A1 dargestellten und beschriebenen Syntheseweg zur Herstellung von Capecitabin erfolgen.

Wie aus Fig. A ersichtlich, wird z.B. Glucose, deren OH-Gruppen mit Acetat-Schutzgruppen entsprechend der Formel (I) versehen sind, in einem organischen Lösungsmittel, beispielsweise Dichlormethan, mit einer mit Trimethylsisyl-Schutzgruppen versehenen Pyrimidin-Base, z.B. Uracil oder Cytosin, die bzw. das entsprechend der Formel (II) in der 5-Position durch Oxymethyl-Furfural substituiert ist, mit Zinntetrachlorid als Katalysator zu trimethylsisyliertem Uracil- oder Cytosin-1N-Glucosid mit acetylierten 2' bis 4' und 6'-OH-Gruppen entsprechend der Formel (III) umgesetzt.

Die mit dem Oxymethyl-Furfural-Rest substitutierte Pyrimidin-Base bzw. Uracil oder Cytosin kann mittels Williamson-Ethersynthese aus dem Alkoholat von 5-Hydroxymethyl-Furfural und beispielsweise 5-Fluor- oder 5-Jod-Uracil oder -Cytosin oder auch unter Verwendung von Silberjodid aus 5-Hydroxymethyl-Furfural und z.B. 5-Fluor oder 5-Jod Uracil oder Cytosin hergestellt werden.

Anschließend werden die Acetyl- und Trimethylsilyl-Schutzgruppen in basischer alkoholischer Lösung z.B. mit 1-normaler Natronlauge in methanolischer Lösung abgespalten, um das 5-Hydroxymethyl-furfural-1'-glucosid von Cytosin (oder Uracil) entsprechend der Formel (IV) zu erhalten.

Statt Glucose kann auch Gluconsäure in ihrer 1'-Position an die N-1-Position der Pyrimidin-Base, also z.B. von Uracil oder Cytosin, gebunden sein.

Die Gluconsäure ist dabei vorzugsweise in ihrer 5'-Position mit Hydroxymethyl-Furfural verestert. Die Esterbildung kann durch Umsetzung des Silbersalzes der Gluconsäure mit Jodmethyl-Furfural unter Abspaltung von Jodid entsprechend DE 10 2013 106 530 A erfolgen.

Falls die in 5'-Position mit Hydroxmethyl-Furfural veresterte Gluconsäure mit ihrer 1'-Position an die N-1-Position der Pyrimidin-Base, also z.B. Uracil oder Cytosin, gebunden ist und die Pyrimidin-Base bzw. Cytosin oder Uracil in der 5'-Position z.B. mit einer Oxymethylfurfural-Gruppe substituiert ist, weist das Krebsmedikament eine Verbindung mit zwei Furfural-Gruppen auf, die eine entsprechend hohe Toxizität gegenüber der Krebszelle besitzt.

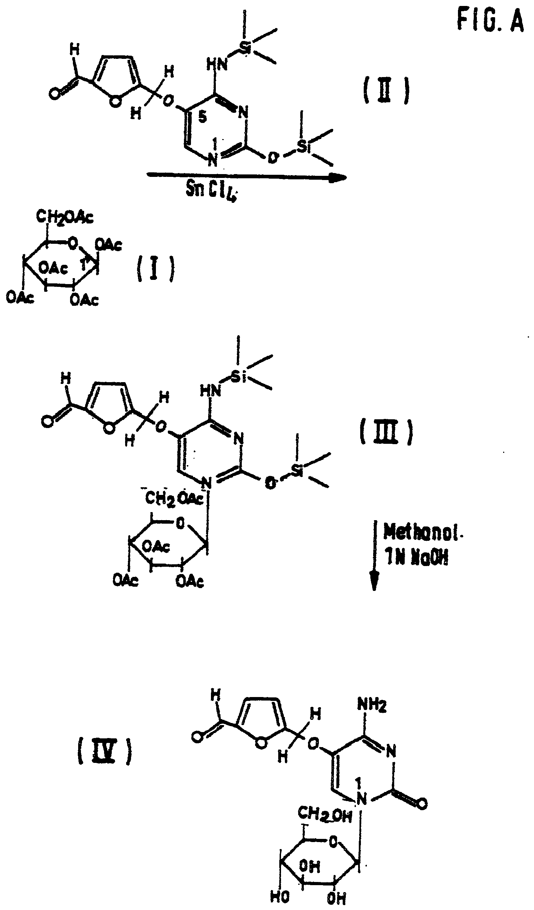

## Patentansprüche

1. Krebsmedikament, das ein Pyrimidin-Derivat enthält, das durch eine Pyrimidin-Base mit einem in N-1-Position glykosidisch gebundenen Zuckerderivat gebildet wird und wenigstens eine Furfural-Gruppe aufweist, **dadurch gekennzeichnet, dass** das Zuckerderivat Glucose oder Gluconsäure ist, die an die N-1-Position der Pyrimidin-Base gebunden ist, welche in ihrer 5-Position mit einer Furfural-Gruppe substituiert ist.

2. Krebsmedikament nach Anspruch 1, **dadurch gekennzeichnet, dass** die Pyrimidin-Base durch eine Ether-Gruppe mit der Furfural-Gruppe verbunden ist.

3. Krebsmedikament nach Anspruch 2, **dadurch gekennzeichnet, dass** die Ether-Gruppe durch eine Furfural-oxymethyl-Gruppe gebildet wird.

4. Krebsmedikament nach einen der vorstehenden Ansprüche, **dadurch gekennzeichnet, dass** die Gluconsäure mit Hydroxymethyl-Furfural verestert ist.

5. Krebsmedikament nach einen der vorstehenden Ansprüche, **dadurch gekennzeichnet, dass** die Pyrimidin-Base durch Uracil oder Cytosin gebildet wird.
